(19) **Europäisches Patentamt / European Patent Office / Office européen des brevets**

(11) **EP 3 778 636 A1**

(12) **EUROPEAN PATENT APPLICATION**
published in accordance with Art. 153(4) EPC

(43) Date of publication:
**17.02.2021 Bulletin 2021/07**

(21) Application number: **19774201.8**

(22) Date of filing: **27.03.2019**

(51) Int Cl.:
*C07K 16/28* (2006.01)  *C12N 15/09* (2006.01)
*C12N 5/10* (2006.01)  *C12N 1/21* (2006.01)
*C12N 1/15* (2006.01)  *C12P 21/08* (2006.01)
*A61K 39/395* (2006.01)  *A61P 35/00* (2006.01)
*A61P 37/00* (2006.01)

(86) International application number:
**PCT/CN2019/079811**

(87) International publication number:
**WO 2019/184935 (03.10.2019 Gazette 2019/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.03.2018 CN 201810267050**

(71) Applicant: **Jiangsu Hengrui Medicine Co., Ltd.**
**Lianyungang, Jiangsu 222047 (CN)**

(72) Inventors:
• **HUANG, Hao**
**Lianyungang, Jiangsu 222047 (CN)**
• **FANG, Yan**
**Lianyungang, Jiangsu 222047 (CN)**

• **YAN, Zhen**
**Lianyungang, Jiangsu 222047 (CN)**
• **SHI, Ruijun**
**Lianyungang, Jiangsu 222047 (CN)**
• **JIANG, Jiahua**
**Lianyungang, Jiangsu 222047 (CN)**
• **CAO, Guoqing**
**Lianyungang, Jiangsu 222047 (CN)**
• **ZHANG, Lianshan**
**Lianyungang, Jiangsu 222047 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **ANTI-CD27 ANTIBODY, ANTIGEN-BINDING FRAGMENT THEREOF AND MEDICAL USE THEREOF**

(57) The present invention provides an anti-CD27 antibody, an antigen-binding fragment thereof, and a medical use thereof. Specifically, the present invention provides a human antibody comprising the CDR region of the anti-CD27 antibody, and a pharmaceutical composition comprising the human anti-CD27 antibody and an antigen-binding fragment thereof, and a use thereof as an anticancer drug. In particular, the invention provides a human anti-CD27 antibody and a use thereof in the preparation of a drug for the treatment of CD27-mediated diseases or disorders.

EP 3 778 636 A1

SEQUENCE LISTING

<110>  JIANGSU HENGRUI MEDICINE CO., LTD.

<120>  ANTI-CD27 ANTIBODY, ANTIGEN-BINDING FRAGMENT THEREOF AND MEDICAL
       USE THEREOF

<130>  P20413513EP

<140>  PCT/CN2019/079811
<141>  2019-03-27

<150>  CN201810267050.4
<151>  2018-03-28

<160>  14

<170>  PatentIn version 3.5

<210>  1
<211>  119
<212>  PRT
<213>  Homo sapiens

<400>  1

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Gly Tyr
            20                  25                  30

Gly Ile His Trp Val Arg Gln Ala Pro Gly Lys Gly Leu Glu Trp Ile
        35                  40                  45

Gly Trp Ile Asn Pro Asn Arg Gly Ser Thr Lys Tyr Ala Gln Lys Phe
    50                  55                  60

Gln Gly Arg Val Thr Ile Ser Arg Asp Asn Ser Lys Asn Thr Leu Tyr
65                  70                  75                  80

Leu Gln Leu Asn Ser Leu Arg Ala Glu Asp Thr Ala Val Tyr Tyr Cys
                85                  90                  95

Ala Arg Asp Pro Gly Tyr Thr Trp Tyr Phe Asp Val Trp Gly Gln Gly
            100                 105                 110

Thr Leu Val Thr Val Ser Ser
            115
```

<210>  2
<211>  107
<212>  PRT
<213>  Homo sapiens

<400> 2

Asp Ile Gln Leu Thr Gln Ser Pro Ser Ser Leu Ser Ala Ser Val Gly
1               5                   10                  15

Asp Arg Val Thr Ile Thr Cys Arg Ala Ser Gln Asp Ile Ser Ser Asp
            20                  25                  30

Leu Ala Trp Tyr Gln Gln Lys Pro Gly Lys Ala Pro Lys Leu Leu Ile
            35                  40                  45

Tyr Ala Ala Ser Thr Leu Gln Ser Gly Val Pro Ser Arg Phe Ser Gly
            50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Arg Asp Ala Trp Pro Pro
                85                  90                  95

Thr Phe Gly Gln Gly Thr Lys Val Glu Ile Lys
            100                 105

<210> 3
<211> 5
<212> PRT
<213> Homo sapiens

<400> 3

Gly Tyr Gly Ile His
1               5

<210> 4
<211> 17
<212> PRT
<213> Homo sapiens

<400> 4

Trp Ile Asn Pro Asn Arg Gly Ser Thr Lys Tyr Ala Gln Lys Phe Gln
1               5                   10                  15

Gly

<210> 5
<211> 10
<212> PRT
<213> Homo sapiens

<400> 5

```
Asp Pro Gly Tyr Thr Trp Tyr Phe Asp Val
1               5               10


<210>   6
<211>   11
<212>   PRT
<213>   Homo sapiens

<400>   6

Arg Ala Ser Gln Asp Ile Ser Ser Asp Leu Ala
1               5               10


<210>   7
<211>   7
<212>   PRT
<213>   Homo sapiens

<400>   7

Ala Ala Ser Thr Leu Gln Ser
1               5


<210>   8
<211>   9
<212>   PRT
<213>   Homo sapiens

<400>   8

Gln Gln Arg Asp Ala Trp Pro Pro Thr
1               5


<210>   9
<211>   214
<212>   PRT
<213>   Homo sapiens

<400>   9

Asp Ile Gln Leu Thr Gln Ser Pro Ser Ser Leu Ser Ala Ser Val Gly
1               5               10              15


Asp Arg Val Thr Ile Thr Cys Arg Ala Ser Gln Asp Ile Ser Ser Asp
            20              25              30


Leu Ala Trp Tyr Gln Gln Lys Pro Gly Lys Ala Pro Lys Leu Leu Ile
            35              40              45


Tyr Ala Ala Ser Thr Leu Gln Ser Gly Val Pro Ser Arg Phe Ser Gly
        50              55              60


Ser Gly Ser Gly Thr Asp Phe Thr Leu Thr Ile Ser Ser Leu Gln Pro
65              70              75              80
```

```
Glu Asp Phe Ala Thr Tyr Tyr Cys Gln Gln Arg Asp Ala Trp Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
            115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
        210
```

```
<210>  10
<211>  449
<212>  PRT
<213>  Homo sapiens

<400>  10
```

```
Glu Val Gln Leu Val Glu Ser Gly Gly Gly Leu Val Gln Pro Gly Gly
1               5               10                  15

Ser Leu Arg Leu Ser Cys Ala Ala Ser Gly Phe Thr Phe Ser Gly Tyr
            20                  25                  30

Gly Ile His Trp Val Arg Gln Ala Pro Gly Lys Gly Leu Glu Trp Ile
            35                  40                  45

Gly Trp Ile Asn Pro Asn Arg Gly Ser Thr Lys Tyr Ala Gln Lys Phe
        50                  55                  60

Gln Gly Arg Val Thr Ile Ser Arg Asp Asn Ser Lys Asn Thr Leu Tyr
65                  70                  75                  80
```

```
Leu Gln Leu Asn Ser Leu Arg Ala Glu Asp Thr Ala Val Tyr Tyr Cys
                85                  90                  95

Ala Arg Asp Pro Gly Tyr Thr Trp Tyr Phe Asp Val Trp Gly Gln Gly
               100                 105                110

Thr Leu Val Thr Val Ser Ser Ala Ser Thr Lys Gly Pro Ser Val Phe
               115                 120                125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
               165                 170                175

Gln Ser Ser Gly Leu Tyr Ser Leu Ser Ser Val Val Thr Val Pro Ser
               180                 185                190

Ser Ser Leu Gly Thr Gln Thr Tyr Ile Cys Asn Val Asn His Lys Pro
               195                 200                205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
               245                 250                255

Arg Thr Pro Glu Val Thr Cys Val Val Val Asp Val Ser His Glu Asp
               260                 265                270

Pro Glu Val Lys Phe Asn Trp Tyr Val Asp Gly Val Glu Val His Asn
               275                 280                285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
               325                 330                335
```

Thr Ile Ser Lys Ala Lys Gly Gln Pro Arg Glu Pro Gln Val Tyr Thr
        340             345             350

Leu Pro Pro Ser Arg Asp Glu Leu Thr Lys Asn Gln Val Ser Leu Thr
        355             360             365

Cys Leu Val Lys Gly Phe Tyr Pro Ser Asp Ile Ala Val Glu Trp Glu
        370             375             380

Ser Asn Gly Gln Pro Glu Asn Asn Tyr Lys Thr Thr Pro Pro Val Leu
385             390             395             400

Asp Ser Asp Gly Ser Phe Phe Leu Tyr Ser Lys Leu Thr Val Asp Lys
                405             410             415

Ser Arg Trp Gln Gln Gly Asn Val Phe Ser Cys Ser Val Met His Glu
        420             425             430

Ala Leu His Asn His Tyr Thr Gln Lys Ser Leu Ser Leu Ser Pro Gly
        435             440             445

Lys

<210> 11
<211> 119
<212> PRT
<213> Artificial Sequence

<220>
<223> 077N74T VH

<400> 11

Glu Val Gln Leu Val Glu Ser Gly Gly Gly Leu Val Gln Pro Gly Gly
1               5               10              15

Ser Leu Arg Leu Ser Cys Ala Ala Ser Gly Phe Thr Phe Ser Gly Tyr
        20              25              30

Gly Ile His Trp Val Arg Gln Ala Pro Gly Lys Gly Leu Glu Trp Ile
        35              40              45

Gly Trp Ile Asn Pro Asn Arg Gly Ser Thr Lys Tyr Ala Gln Lys Phe
        50              55              60

Gln Gly Arg Val Thr Ile Ser Arg Asp Thr Ser Lys Asn Thr Leu Tyr
65              70              75              80

```
Leu Gln Leu Asn Ser Leu Arg Ala Glu Asp Thr Ala Val Tyr Tyr Cys
                85                  90                  95

Ala Arg Asp Pro Gly Tyr Thr Trp Tyr Phe Asp Val Trp Gly Gln Gly
            100                 105                 110

Thr Leu Val Thr Val Ser Ser
            115


<210>   12
<211>   449
<212>   PRT
<213>   Artificial Sequence

<220>
<223>   077N74T HC

<400>   12

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Gly Tyr
            20                  25                  30

Gly Ile His Trp Val Arg Gln Ala Pro Gly Lys Gly Leu Glu Trp Ile
            35                  40                  45

Gly Trp Ile Asn Pro Asn Arg Gly Ser Thr Lys Tyr Ala Gln Lys Phe
            50                  55                  60

Gln Gly Arg Val Thr Ile Ser Arg Asp Asn Ser Lys Asn Thr Leu Tyr
65                  70                  75                  80

Leu Gln Leu Asn Ser Leu Arg Ala Glu Asp Thr Ala Val Tyr Tyr Cys
                85                  90                  95

Ala Arg Asp Pro Gly Tyr Thr Trp Tyr Phe Asp Val Trp Gly Gln Gly
            100                 105                 110

Thr Leu Val Thr Val Ser Ser Ala Ser Thr Lys Gly Pro Ser Val Phe
            115                 120                 125

Pro Leu Ala Pro Ser Ser Lys Ser Thr Ser Gly Gly Thr Ala Ala Leu
            130                 135                 140

Gly Cys Leu Val Lys Asp Tyr Phe Pro Glu Pro Val Thr Val Ser Trp
145                 150                 155                 160
```

```
Asn Ser Gly Ala Leu Thr Ser Gly Val His Thr Phe Pro Ala Val Leu
            165             170             175

Gln Ser Ser Gly Leu Tyr Ser Leu Ser Ser Val Val Thr Val Pro Ser
            180             185             190

Ser Ser Leu Gly Thr Gln Thr Tyr Ile Cys Asn Val Asn His Lys Pro
            195             200             205

Ser Asn Thr Lys Val Asp Lys Lys Val Glu Pro Lys Ser Cys Asp Lys
            210             215             220

Thr His Thr Cys Pro Pro Cys Pro Ala Pro Glu Leu Leu Gly Gly Pro
225             230             235             240

Ser Val Phe Leu Phe Pro Pro Lys Pro Lys Asp Thr Leu Met Ile Ser
            245             250             255

Arg Thr Pro Glu Val Thr Cys Val Val Val Asp Val Ser His Glu Asp
            260             265             270

Pro Glu Val Lys Phe Asn Trp Tyr Val Asp Gly Val Glu Val His Asn
            275             280             285

Ala Lys Thr Lys Pro Arg Glu Glu Gln Tyr Asn Ser Thr Tyr Arg Val
            290             295             300

Val Ser Val Leu Thr Val Leu His Gln Asp Trp Leu Asn Gly Lys Glu
305             310             315             320

Tyr Lys Cys Lys Val Ser Asn Lys Ala Leu Pro Ala Pro Ile Glu Lys
            325             330             335

Thr Ile Ser Lys Ala Lys Gly Gln Pro Arg Glu Pro Gln Val Tyr Thr
            340             345             350

Leu Pro Pro Ser Arg Asp Glu Leu Thr Lys Asn Gln Val Ser Leu Thr
            355             360             365

Cys Leu Val Lys Gly Phe Tyr Pro Ser Asp Ile Ala Val Glu Trp Glu
            370             375             380

Ser Asn Gly Gln Pro Glu Asn Asn Tyr Lys Thr Thr Pro Pro Val Leu
385             390             395             400

Asp Ser Asp Gly Ser Phe Phe Leu Tyr Ser Lys Leu Thr Val Asp Lys
            405             410             415
```

```
Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
            435                 440                 445

Lys


<210>  13
<211>  98
<212>  PRT
<213>  Homo sapiens

<400>  13

Glu Val Gln Leu Val Glu Ser Gly Gly Gly Leu Val Gln Pro Gly Gly
1               5               10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Val Trp Val
            35                  40                  45

Ser Arg Ile Asn Ser Asp Gly Ser Ser Thr Ser Tyr Ala Asp Ser Val
        50                  55                  60

Lys Gly Arg Phe Thr Ile Ser Arg Asp Asn Ala Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg


<210>  14
<211>  95
<212>  PRT
<213>  Homo sapiens

<400>  14

Asp Ile Gln Met Thr Gln Ser Pro Ser Ser Val Ser Ala Ser Val Gly
1               5               10                  15

Asp Arg Val Thr Ile Thr Cys Arg Ala Ser Gln Gly Ile Ser Ser Trp
            20                  25                  30
```

```
Leu Ala Trp Tyr Gln Gln Lys Pro Gly Lys Ala Pro Lys Leu Leu Ile
        35                  40              45


Tyr Ala Ala Ser Ser Leu Gln Ser Gly Val Pro Ser Arg Phe Ser Gly
        50              55                  60


Ser Gly Ser Gly Thr Asp Phe Thr Leu Thr Ile Ser Ser Leu Gln Pro
65              70                  75                      80


Glu Asp Phe Ala Thr Tyr Tyr Cys Gln Gln Ala Asn Ser Phe Pro
                85                  90                  95
```

**Description**

The present application claims priority to Chinese patent application NO: CN201810267050.4 filed on March28th, 2018,the content of which is incorporated herein by reference in its entirety.

**Technical Field**

The present invention relates to an anti-CD27 antibody, an antigen-binding fragment of CD27, a chimeric antibody and a humanized antibody comprising CDR regions of the antibody, and a pharmaceutical composition comprising human anti-CD27 antibody and antigen-binding fragment thereof, and a use thereof as an anti-cancer drug.

**Background**

Tumor immunotherapy is a long-term hot spot in the field of anti-cancer drugs. It makes full use of and motivates the Killer T cell in tumor patients to kill and destroy the tumor. It may be one of the most effective and safest tumor treatment methods. The activation of T cells in the human body is a system with two signal pathways. In addition to the MHC antigen peptides presented by antigen-presenting cells as the first signal which is provided to T cells, it also requires a series of co-stimulatory molecules to provide the second signal. Both of pathways enable T cells to trigger a normal immune response.

The interaction between T cells and antigen-presenting cells consists of a variety of helper molecules that conduce to trigger immune response, and CD27 is one of these molecules. CD27 belongs to the tumor necrosis factor receptor (TNF-R) superfamily and binds to CD70 on the surface of T cells. CD27 is a glycosylated type I transmembrane protein, which is usually a homodimer connected by disulfide bridges. The disulfide bridges are located in the area near the cell membrane in the extracellular domain (Camerini et al., J Immunol. 147: 3165-69, 1991). On T cells, cross-linked CD27 antigen can provide a synergistic stimulation signal, and when cross-linked with T cell receptors, it can induce T cell proliferation and cellular immune activation.

CD27 is expressed in mature thymocytes, most CD4[+] and CD8[+] external blood T cells, natural killer cells and B cells, and is also highly expressed in B-cell non-Hodgkin's lymphoma and B-cell chronic lymphocytic leukemia; and in infections of parasites and cytomegalovirus (CMV) and the like, multiple sclerosis, sarcoidosis, and B-cell chronic lymphocytic leukemia, improved levels of soluble CD27 protein have also been observed in serum or in active parts of the disease (Kobata T et al., Proc. Natl. Acad. Sci. USA. 1995 (24): 11249-53; Ranheim EA et al., Blood. 1995 85 (12): 3556-65; Loenen WA et al., Eur. J. Immunol. 1992 22: 447).

Recent studies have shown that anti-CD27 monoclonal antibody agonists can promote T cell responses and can be used as potential anti-cancer therapeutic agent (see patents WO2008051424, WO2011130434, PCT/CN2017/103842). Although the research results obtained so far have identified CD27 as a useful target for immunotherapy, the specific characteristics of anti-CD27 monoclonal antibodies that are particularly beneficial for treatment remain to be ascertained. It is still necessary to further understand which specific functional properties of anti-CD27 monoclonal antibodies are therapeutically effective in the art, so as to further obtain potentially improved anti-CD27 antibodies that are more effective in preventing and treating diseases.

At present, many international companies have developed tumor antibody drugs against the CD27 target. Anti-CD27 antibody from US Celldex is already in phase II clinical trials, while relevant products from companies such as Merck, Aduro and Apogenix are still in preclinical development.

Therefore, there is a need in the art to provide an anti-CD27 antibody with high affinity, high selectivity, high biological activity and high stability, and the antibody with specific functional properties that can be correlated with the desired beneficial therapeutic effect.

**Content of the present invention**

Some embodiments of the present invention provide an anti-CD27 antibody or an antigen-binding fragment thereof, comprising: a light chain variable region and a heavy chain variable region, wherein the light chain variable region of the antibody comprises amino acid sequences of LCDR1 shown in SEQ ID NO: 6, LCDR2 shown in SEQ ID NO: 7 and/or LCDR3 shown in SEQ ID NO: 8, respectively; and/or, the heavy chain variable region of the antibody comprises amino acid sequences of HCDR1 shown in SEQ ID NO: 3, HCDR2 shown in SEQ ID NO: 4, and/or HCDR3 shown in SEQ ID NO: 5, respectively.

Some embodiments provide an anti-CD27 antibody or an antigen-binding fragment thereof, comprising: a light chain variable region and a heavy chain variable region, wherein the light chain variable region of the antibody comprises amino acid sequences of LCDR1, LCDR2 and LCDR3 shown in SEQ ID NO: 6, SEQ ID NO: 7 and SEQ ID NO: 8, respectively; and/or, the heavy chain variable region of the antibody comprises amino acid sequences of HCDR1, HCDR2 and HCDR3 shown in SEQ ID NO: 3, SEQ ID NO: 4 and SEQ ID NO: 5, respectively.

In some embodiments, the anti-CD27 antibody or the antigen-binding fragment thereof as defined above is a murine antibody, a chimeric antibody, a human antibody, a humanized antibody or a fragment thereof.

In some embodiments, the anti-CD27 antibody or antigen-binding fragment thereof as defined above further comprises Vκ1 sequence framework region of human germline light chain variable region or a derivative sequence thereof, in at least one embodiment, the framework region of the light chain variable region is derived from the sequence of Vκ1-12; and/or, the anti-CD27 antibody or the antigen-binding fragment thereof further comprises $V_H3$ sequence framework region of human germline heavy chain variable region or a derivative sequence thereof; in at least one embodiment, the framework region of the heavy chain variable region sequence is derived from $V_H3$-74 sequence.

In some embodiments, an asparagine at position 73 according to Kabat in the framework region of the $V_H3$ sequence of the heavy chain variable region of the anti-CD27 antibody or the antigen-binding fragment thereof as defined above is substituted by a threonine.

In some embodiments, the heavy chain of the humanized antibody as defined above comprises a heavy chain constant region of human IgG1, IgG2, IgG3 or IgG4 or a variant thereof, and the antigen-binding fragment is a Fab, Fab'-SH, Fv, scFv and/or (Fab')$_2$ fragment. In at least one embodiment, the heavy chain of the humanized antibody comprises a heavy chain constant region of human IgG1, IgG2 or IgG4.

In some embodiments, the amino acid sequence of the light chain variable region of the anti-CD27 antibody or the antigen-binding fragment thereof is shown in SEQ ID NO: 2, or has at least 90%, 92%, 94%, 95%, 96%, 97%, 98% or 99% identity with SEQ ID NO: 2; and/or the amino acid sequence of the heavy chain variable region sequence is shown in SEQ ID NO: 1 or SEQ ID NO: 11, or has at least 90%, 92%, 94%, 95%, 96%, 97%, 98% or 99% identity with SEQ ID NO: 1 or SEQ ID NO: 11. In at least one embodiment, the amino acid sequence of the light chain of the anti-CD27 antibody or antigen-binding fragment thereof as defined above is shown in SEQ ID NO: 9 or has at least 90%, 92%, 94%, 95%, 96%, 97%, 98% or 99% identity with SEQ ID NO: 9; and/or the amino acid sequence of the heavy chain of the anti-CD27 antibody or antigen-binding fragment thereof as defined above is shown in SEQ ID NO: 10 or SEQ ID NO : 12, or has at least 90%, 92%, 94%, 95%, 96%, 97%, 98% or 99% identity with SEQ ID NO: 10 or SEQ ID NO: 12.

Some embodiments provide an anti-CD27 antibody or an antigen-binding fragment thereof, which comprises: a light chain variable region and a heavy chain variable region, wherein the light chain variable region of the antibody comprises LCDR1, LCDR2 and LCDR3 shown in SEQ ID NO: 6,SEQ ID NO: 7 and SEQ ID NO: 8,respectively. The anti-CD27 antibody or the antigen-binding fragment thereof comprises the framework region of Vκ1 sequence of human germline light chain variable region; the heavy chain variable region of the antibody comprises HCDR1, HCDR2 and HCDR3 shown in SEQ ID NO: 3, SEQ ID NO: 4 and SEQ ID NO:5,respectively,and the framework region of $V_H3$ sequence of human germline heavy chain variable region.

In at least one embodiment, the framework region of the light chain variable region of the anti-CD27 antibody is selected from the framework region derived from Vκ1-12 sequence; the framework region of the heavy chain variable region is selected from the framework region derived from $V_H3$-74sequence.

The sequence of human germline $V_H3$-74 (with IMGT accession number IGHV3-74*01) is provided as follows:

EVQLVESGGGLVQPGGSLRLSCAASGFTFSSYWMHWVRQAPGKGLVWV
SRINSDGSSTSYADSVKGRFTISRDNAKNTLYLQMNSLRAEDTAVYYCAR

SEQ ID NO: 13

The sequence of human germline Vκ1-12 (with IMGT accession number IGKV1-12*01) is provided as follows:

DIQMTQSPSSVSASVGDRVTITCRASQGISSWLAWYQQKPGKAPKLLIYA
ASSLQSGVPSRFSGSGSGTDFTLTISSLQPEDFATYYCQQANSFP

SEQ ID NO: 14

In at least one embodiment, an asparagine (Asn) at position 73 according to Kabat in the framework region sequence of the VH3 sequence of the heavy chain variable region of the antibody is substituted by a threonine (Thr).

In at least one embodiment, the sequence of light chain variable region of the anti-CD27 antibody is SEQ ID NO:1, and the sequence of heavy chain variable region is SEQ ID NO:11.

In at least one embodiment, the anti-CD27 antibody or the antigen-binding fragment thereof is a human antibody or fragment thereof.

In at least one embodiment, the light chain sequence of the anti-CD27 antibody is: SEQ ID NO: 9; and the heavy chain sequence of the antibody is: SEQ ID NO: 12.

Some embodiments provide an anti-CD27 antibody or antigen-binding fragment thereof, which comprises: a light chain variable region and a heavy chain variable region, wherein the sequence of light chain variable region of the antibody is SEQ ID NO:1, and the sequence of heavy chain variable region of the antibody is SEQ ID NO: 11.

In at least one embodiment, the light chain sequence of the anti-CD27 antibody is SEQ ID NO: 9; and the antibody heavy chain sequence is: SEQ ID NO: 11.

Some embodiments provide an isolated monoclonal antibody or an antigen-binding fragment that can compete with the monoclonal antibody or the antigen-binding fragment as defined above for binding to CD27.

Some embodiments provide a multi specific antibody, which comprises the light chain variable region and the heavy chain variable region as defined above.

Some embodiments provide a single chain antibody, which comprises the light chain variable region and the heavy chain variable region as defined above.

Some embodiments provide an antibody-drug conjugate, which comprises the light chain variable region and the heavy chain variable region as defined above. The antibody-drug conjugate is well-known in the art and are formed by the interconnection of antibody-linker-drug (toxin). Known linkers include cleavage linkers and non-cleavage linkers. For example, linkers include but are not limited to SMCC, SPDP and the like; toxins are also well known in the art, such as DM1, DM4, MMAE, MMAF and the like.

Some embodiments provide a nucleic acid encoding the anti-CD27 antibody or the antigen-binding fragment as defined above.

Some embodiments provide an expression vector containing the nucleic acid as defined above.

Some embodiments provide a host cell transformed with an expression vector as defined above.

In at least one embodiment, the host cell as defined above which is a bacteria, preferably *Escherichia coli.*

In at least one embodiment, the host cell as defined above which is a yeast, preferably *Pichia pastoris.*

In at least one embodiment, a host cell as defined above which is a mammalian cell, preferably a Chinese hamster ovary (CHO) cell or a human embryonic kidney (HEK) 293 cell.

Some embodiments provide a method for preparing an anti-CD27 antibody and an antigen-binding fragment thereof, which comprises expressing the antibody or antigen-binding fragment thereof in the host cell as defined above and isolating the antibody or antigen-binding fragment from the host cell.

Some embodiments provide a pharmaceutical composition, which comprises the anti-CD27 antibody or the antigen-binding fragment thereof as defined above and a pharmaceutically acceptable excipient, dilution or carrier.

Some embodiments provide a use of the anti-CD27 antibody or antigen-binding fragment thereof, or a pharmaceutical composition comprising thereof as defined above in the preparation of a medicament for the treatment and/or prevention of a CD27-mediated disease or disorder; wherein the disease defined therein is preferably a cancer and an autoimmune disease; more preferably a cancer or an autoimmune disease withCD27 expression; the cancers are most preferably rectal cancer, brain cancer, renal cancer, lung cancer, liver cancer, multiple myeloma and melanoma; the autoimmune diseases is most preferably autoimmune encephalomyelitis, systemic lupus erythematosus, multiple sclerosis and rheumatoid arthritis.

Some embodiments provide a method for the treatment and/or prevention of a CD27-mediated disease or disorder, the method comprises administering to a patient in need a therapeutically effective amount of the anti-CD27 antibody or the antigen-binding fragment thereof as defined above, or the pharmaceutical composition comprising the anti-CD27 antibody or antigen-binding fragment thereof as defined above, wherein the disease is preferably a cancer or an autoimmune disease; more preferably a cancer or an autoimmune disease with CD27expression; the cancer is most preferably rectal cancer, brain cancer, renal cancer, lung cancer, liver cancer, multiple myeloma and melanoma; the autoimmune disease is most preferably autoimmune encephalomyelitis, systemic lupus erythematosus, multiple sclerosis and rheumatoid arthritis.

**Brief description of the drawings**

Figure 1: The deamidation status of the acidic component and main peak component of mAb077was identified by chromatography-mass spectrometry. The results showed that the acidic component was mainly deamidated 74th amino acid asparagine (Asn), and the reaction ratio reached59.6%.

Figure 2: The acid-base peak distribution status of mAb077 before mutation and 077N74T after mutation was identified by SEC-HPLC method. An acidic peak was eluted before the main peak at 29 minutes in mAb077.

Figure 3: After heat treatment at 40°C for 1 month, the distribution of 077N74T acid-base isoelectric point components and changes relative to the initial state thereof were measured using ICE.

Figure 4: Different concentrations of anti-CD27 antibody (pre/post mutation molecule) or Herceptin(use as a reference)was used to stimulate the functional strength of human blood PBMC proliferation.

Figure 5: Tumor size(indicated as mm3) of human Raji transplant lymphoma in mice treated with control human IgG1 antibody or various anti-CD27 antibodies relative to the number of days after tumor inoculation. The error indicates the standard error.

Figure 6: Changes(indicated as g)in body weight of mice treated with control human IgG1 antibody or various anti-CD27 antibodies. The error indicates the standard error.

**Detailed description of the present invention**

1. Definition

Some technical and scientific terms are specifically defined below. Unless otherwise clearly defined elsewhere in this document, all other technical and scientific terms used herein have the meanings commonly understood by those of ordinary skill in the art to which the present invention belongs.

The three-letter amino acid code and single-letter code used herein are described in J. Biol. Chem, 243, p3558 (1968).

The term 'antibody' as used herein refers to an immunoglobulin, which is a tetrapeptide chain structure formed by linking two identical heavy chains and two identical light chains by interchain disulfide bonds. As the heavy chain constant regions of immunoglobulins have different composition and order of amino acids, the antigenicity of immunoglobulins is different. Accordingly, immunoglobulins can be classified into five classes, or be referred to as the isotypes of immunoglobulins, namely IgM, IgD, IgG, IgA and IgE, and the corresponding heavy chains of which are $\mu$ chain, $\delta$ chain, $\gamma$ chain, $\alpha$ chain and $\epsilon$ chain, respectively. The same type of Ig can be divided into different subclasses according to the difference in the amino acid composition of the hinge region and the number and position of heavy chain disulfide bonds. For example, IgG can be classified into IgG1, IgG2, IgG3 and IgG4. Light chains can be classified as $\kappa$ chain or $\lambda$ chain according to the differences in their constant regions. Each of the five classes of Ig may have a $\kappa$ chain or a $\lambda$ chain.

The antibody light chain can further comprise a light chain constant region, which comprises a human or murine $\kappa$, $\lambda$ chain or variants thereof.

The antibody heavy chain can further comprise a heavy chain constant region, which comprises a human or murine IgG1, IgG2, IgG3, IgG4 or variants thereof.

Variable region (V region), composed of amino acids close to the N-terminus of the antibody heavy and light chains, varies considerably in its amino acid sequence. Constant region (C region), composed of the remaining amino acid sequence close to the C-terminus of the antibody, is relatively stable. The variable regions of the light and heavy chains are composed of about 110 amino acids, and the changes of amino acid residue in some regions, such as positions 24-34, 50-56, 89-97 in the light chain and positions 31-35, 50-65, 95-102 in the heavy chain, are more frequent than other parts of the variable region. These regions are called hypervariable regions (HVR). Because the hypervariable regions are the parts where antibodies and epitopes directly contact, they are also called complementarity-regions (CDR). The non-hypervariable region in the variable region has relatively fewer changes in amino acid composition and sequence, and these amino acid residues constitute a stable three-dimensional structure of the variable region, namely the framework structure or the framework region (FR). The variable region includes 3 hypervariable regions (HVR) and 4 framework regions (FR) with relatively conservative sequences. Each light chain variable region (VL) and heavy chain variable region (VH) is composed of 3 CDR regions and 4 FR regions. The order of these regions from the amino terminal to the carboxy terminal is: FR1, CDR1, FR2, CDR2, FR3, CDR3 and FR4. The 3 CDR regions of the light chain refer to LCDR1, LCDR2 and LCDR3; the 3 CDR regions of the heavy chain refer to HCDR1, HCDR2 and HCDR3. According to the information of the Kabat database, the amino acid numbers of each regions of the antibody molecule are as follows:

| V region | FR1 | CDR1 | FR2 | CDR2 | FR3 | CDR3 | FR4 |
| --- | --- | --- | --- | --- | --- | --- | --- |
| H | 1-30 | 31-35 | 36-49 | 50-65 | 66-94 | 95-102 | 103-113 |
| L | 1-23 | 24-34 | 35-49 | 50-56 | 57-88 | 89-97 | 98-107 |

The number and position of CDR amino acid residues of the VL and VH regions of the antibodies or antigen binding fragments of the present invention comply with known Kabat numbering criteria.

In the present disclosure, an *in vitro* phagemid display system was used for the screening of human antibody fragments targeting human CD27 antigen, and a series of anti-CD27 antibodies were identified through the screening, wherein the heavy chain variable region of SEQ ID NO:1 and the light chain variable region of SEQ ID NO: 2were selected for further research. Among them, the SEQ ID NO:1 sequence is derived from the $V_H3$ family, which is most similar to the $V_H3$-74 germline sequence. The SEQ ID NO: 2 sequence is derived from the $V\kappa1$ family, which is most similar to the $V\kappa1$-12 germline sequence.

The $V_H3$ family is part of components of the human VH germline. The components of the VH germline can be divided into 7 families based on homology of nucleotide sequences, namely VH1-VH7 (Tomlinson et al. (1992) J. Mol. Biol., 227, 776-798 and Cook et al. (1995) Immunology Today, 16, 237-242). For any given human $V_H3$ germline antibody sequence, the amino acid sequence identity is the highest in the entire $V_H3$ family (see, for example, Tomlinson et al. (1992) J. Mol. Biol., 227, 776-798 and Cook et al. (1995) Immunology Today, 16, 237-242). The amino acid sequence identity between any two $V_H3$ family germline VH sequences varies from 69 to 98 residues of 100 VH residues (i.e. the amino acid sequence homology between any two germline VH sequences is 69-98%). For most germline sequence pairs, there is at least 80 or more identical amino acid residues in 100 amino acid residues (i.e., at least 80% amino acid sequence homology).

The $V\kappa1$ family is a part of all components of the human $V\kappa$ germline. All components of the $V\kappa$ germline can be divided into 7 families based on homology of nucleotide sequences, namely $V\kappa1$-$V\kappa7$ (Enrique et al. (1997) Int Immunol. Dec;9 (12):1801-15.). For most germline sequence pairs, there is at least 80% amino acid sequence homology among $V\kappa$ sequences of $V\kappa1$ family germline.

In view of the high amino acid sequence similarity and structural similarity among $V_H3$ family sequences, those skilled in the art can use chain shuffling technology (Winter et al. (1994) Annual Rev. Immunol., 12, 433-55) or graft the CDRs of rodent antibodies or other human antibodies (including the CDR of the antibodies of the present invention) onto the framework region of the $V_H3$ family and select a suitable VL to pair with it. Similarly, the $V\kappa1$ family sequence also share high amino acid sequence similarity and structural similarity, those skilled in the art can use chain shuffling technology (Winter et al. (1994) Annual Rev. Immunol., 12, 433-55) or graft the CDRs of rodent antibodies or other human antibodies (including the CDR of the antibodies of the present invention) onto the framework region of the $V\kappa1$ family, and select a suitable VH to pair with it.

The term 'antigen presenting cell' or 'APC' is a cell that displays a foreign antigen complexed with MHC on its surface. T cells recognize this complex through the T cell receptor (TCR). Examples of APCs include, but are not limited to, dendritic cells (DC), peripheral blood mononuclear cells (PBMC), monocytes, B lymphoblasts, and monocyte-derived dendritic cells (DC). The term 'antigen presentation' refers to the process by which APCs capture antigens and enable them to be recognized by T cells, for example as a component of MHC-I/MHC-II conjugates.

The term 'CD27' refers to cell surface receptor that is a member of the TNF receptor superfamily. CD 27 is a molecule required for the production and long-term maintenance of T cell immunity and play a key role in regulating B cell activation and immunoglobulin synthesis. The term 'CD27' includes any variant or isoform of CD27 that is naturally expressed by a cell(e.g., human CD27 registered in GENBANK with accession number of AAH12160. 1). The antibodies of the invention can be cross-reactive with CD27 from non-human species. Alternatively, the antibody may be human CD27-specific and may not exhibit cross-reactivity with other species. CD27, or any variant or isoform thereof, can be isolated from cells or tissues in which they are naturally expressed, or produced by recombinant techniques using techniques common in the art and described herein. Preferably, the anti-CD27 antibody targets human CD27 with a normal glycosylation pattern.

The term 'human antibody' includes antibodies having variable and constant regions of human germline immunoglobulin sequences. The human antibody of the invention may include amino acid residues which were not encoded by human germline immunoglobulin sequences (e.g., mutations introduced by random or site-specific mutagenesis *in vitro* or by somatic mutation *in vivo*). However, the term 'human antibody' does not include an antibody in which CDR sequence derived from the germline of another mammalian species, such as mouse, has been grafted onto human framework sequences (i.e., 'humanized antibody').

The term 'humanized antibody', also known as CDR-grafted antibody, refers to an antibody produced by grafting a CDR sequence of a murine into the frameworks of a human antibody variable region. It can overcome the intense immune response induced by chimeric antibody carrying a large amount of mouse protein components. To avoid a decrease in activity caused by reducing the immunogenicity, the human antibody variable region can be subjected to minimal reverse mutation to maintain the activity.

The term 'chimeric antibody' is an antibody formed by fusing a variable region of a murine antibody with a constant region of a human antibody, which can alleviate the immune response induced by a murine antibody. To construct the chimeric antibody, hybridoma that secretes murine-specific monoclonal antibody is first constructed and selected, then the variable region gene is cloned from the murine hybridoma cell. Subsequently, the constant region gene of the human antibody is cloned as needed. The murine variable region gene and the human constant region gene are ligated into a chimeric gene and then inserted into a human vector, and finally the chimeric antibody molecule is expressed in the eukaryotic or prokaryotic industrial system. The constant region of human antibody may be selected from the heavy chain constant region of human IgG1, IgG2, IgG3 or IgG4 or variants thereof, preferably comprising heavy chain constant region of human IgG2 or IgG4, or selected from IgG1 without ADCC (antibody-dependent cell-mediated cytotoxicity) toxicity after amino acid mutation.

' Antigen-binding fragments' refers to a Fab fragment, Fab' fragment, $F(ab')_2$ fragment and sFV Fragment of Fv fragment that binds to human CD27, which have antigen-binding activity; comprising one or more CDRs of the antibody of the present invention selected from SEQ ID NO: 3 to SEQ ID NO: 8. The Fv fragment is the smallest antibody fragment that comprises the heavy chain variable region and the light chain variable region of the antibody, but has no constant regions, and has all antigen binding sites. Generally, Fv antibodies also comprises a polypeptide linker between the VH and VL domains and can form the structure required for antigen binding. Different linkers can also be used to connect the two variable regions of antibodies into a polypeptide chain, which is called single chain antibody or single chain Fv (sFv). The term 'binding to CD27' in the present invention refers to the ability to interact with human CD27. The term 'antigen-binding site' of the present invention refers to a discrete three-dimensional site on the antigen that is recognized by the antibody or antigen-binding fragment of the present invention.

The term 'multi-specific antibody' is used in its broadest sense to encompass antibodies having multi-epitopes specificity. These multi-specific antibodies include, but are not limited to, antibodies comprising a heavy chain variable region (VH) and a light chain variable region (VL), wherein the VH-VL unit has multi-epitopes specificity; antibodies having two or more VL and VH regions, each VH-VL unit binding to a different targets or different epitopes of the same target; antibodies having two or more single variable regions, each single variable region binding to different targets or different epitopes of the same target; full-length antibodies, antibody fragments, diabodies, bispecific diabodies and triabodies, antibody fragments that are covalently or non-covalently linked together and the like.

The term 'antibody-drug conjugate' (ADC) refers to an antibody or antibody fragment conjugated to one or more heterologous chemically synthesized molecules, including but not limited to antibody or antibody fragments conjugated to cytotoxic agents.

The term 'single-chain antibody' is a single-chain recombinant protein formed by connecting a heavy chain variable region (VH) and a light chain variable region (VL) of an antibody via a linker peptide, which is the smallest antibody fragment with complete antigen binding sites.

The term 'epitope' refers to a site on an antigen that specifically binds to an immunoglobulin or antibody. An epitope can be formed by adjacent amino acids, non-adjacent amino acid juxtaposed by tertiary folding of a protein. Epitopes formed by adjacent amino acids are typically maintained after exposure to denaturing solvents, while epitopes formed by tertiary folding are typically lost after treatment with denaturing solvents. Epitopes typically include at least 3-15 amino acids in a unique spatial conformation. Methods for determining which epitopes are bound by a given antibody are well known in the art, including immunoblotting and immunoprecipitation assays, and the like. Methods for determining the spatial conformation of epitopes include techniques in the art and techniques described herein, such as X-ray crystallography and two-dimensional nuclear magnetic resonance.

The terms 'specifically bind' or 'selectively bind' refer to the binding of an antibody to an epitope on a predetermined antigen. Typically, when recombinant human CD27 is used as an analyte and an antibody is used as a ligand, the antibody binds to a predetermined antigen with an equilibrium dissociation constant ($K_D$) ofless than about $10^{-7}$ M or even less when measured by surface plasmon resonance (SPR) techniques in an instrument, and its affinity for binding to a predetermined antigen is at least twice its affinity for binding to a non-specific antigen (such as BSA, etc.)other than a predetermined antigen or a closely related antigen. The term 'antibody(ies)that recognize(s) an antigen' can be used interchangeably herein with the term 'antibody(ies) that specifically bind(s)'.

The term 'competitive binding' refers to an antibody that recognizes the same epitope (also called an antigenic determinant) or a part of the same epitope on the extracellular region of human CD27 with the monoclonal antibody of the present invention and binds to the antigen. The antibody that binds to the same epitope as the monoclonal antibody of the present invention refers to an antibody that recognizes and binds to the amino acid sequence of human CD27 recognized by the monoclonal antibody of the present invention.

The term 'nucleic acid molecule' refers to a DNA molecule and a RNA molecule. The nucleic acid molecule can be single-stranded or double-stranded but is preferably a double-stranded DNA. The nucleic acid is 'operatively linked' when a nucleic acid is placed in a functional relationship with another nucleic acid sequence. For example, if a promoter or enhancer affects the transcription of a coding sequence, then the promoter or enhancer is operatively linked to the coding sequence.

The term 'cross-reaction' refers to the ability of an antibody of the present invention to bind to CD27 derived from different species. For example, an antibody of the present invention that binds to human CD27 can also bind to CD27 of another species. Cross-reactivity is measured by detecting specific reactivity of antibodies with purified antigens in binding assays (e.g., SPR and ELISA), or binding or functional interactions of antibodies with cells that express CD27 physiologically. Methods for determining cross-reactivity include standard binding assays, as described herein, such as surface plasmon resonance (SPR) assay or flow cytometry.

The terms 'inhibition' or 'blockade' are used interchangeably and encompass both partial and complete inhibition/blockade. The inhibition/blockade of the CD70 preferably reduces or alters the normal level or type of activity ofCD70 binding without being inhibited or blocked. Inhibition and blockade are also intended to include any measurable reduction in CD70binding affinity when contacted with an anti-CD27 antibody as compared to a CD70that are not contacted with an anti-CD27 antibody.

The term 'inhibition of growth' (e.g., involving cells) is intended to include any measurable reduction in cell growth.

The terms 'inducing immune response' and 'enhancing immune response' can be used interchangeably and refer to the stimulation (i.e., passive or adaptive) of an immune response to a particular antigen. The term 'inducing' specific for inducing CDC or ADCC refers to stimulating specific direct cell killing mechanism.

The 'ADCC', that is, antibody-dependent cell-mediated cytotoxicity, means that cells expressing Fc receptors directly kills target cells coated with antibodies by recognizing Fc segment of the antibody. The ADCC effector function of the antibody can be reduced or eliminated by modification of the Fc fragment of IgG. The modification refers to mutations in the heavy chain constant region of the antibody, such as mutations selected from the group consisting of N297A, L234A, L235A of IgG1; IgG2/4 chimera, F235E of IgG4, and L234A/E235A mutation.

Methods for producing and purifying antibodies and antigen binding fragments are well known and can be found in the prior art, such as the Using Antibodies: A Laboratory Manual, Chapters 5-8 and 15, Cold Spring Harbor. For example, a mouse can be immunized with human CD40 or a fragment thereof, and the resulting antibody can be renatured, purified, and subjected to amino acid sequencing by a conventional method. The antigen binding fragment can also be prepared by a conventional method. The antibodies or antigen binding fragments of the present invention are genetically engineered to introduce one or more human FR regions in a non-human CDR region. Human germline FR sequences are available on the website of ImMunoGeneTics (IMGT) *http://imgt.cines.fr* or from The Immunoglobulin FactsBook, 2001 ISBN 014441351.

The engineered antibodies or antigen binding fragments can be prepared and purified by conventional methods. The cDNA sequence of the corresponding antibody can be cloned and recombined into a GS expression vector. CHO cells can be stably transfected by the recombinant immunoglobulin expression vector. As a more recommended method well known in the art, mammalian expression systems will result in glycosylation of antibodies, particularly at the highly conserved N-terminus of the FC region. Stable clones are obtained by expressing antibodies that specifically bind to human antigens. Positive clones were expanded in serum-free medium in a bioreactor to produce antibodies. Culture medium, into which the antibody is secreted, can be purified and collected by conventional techniques. The antibody can be concentrated by filtration in a conventional manner. Soluble mixtures and multimers can also be removed by conventional methods such as molecular sieves, ion exchange. The resulting product needs to be frozen immediately, such as -70 °C, or lyophilized.

The antibody refers to a monoclonal antibody. The monoclonal antibody (mAb) refers to an antibody obtained from a single clonal cell line, and the cell line is not limited to a eukaryotic, prokaryotic or phage clonal cell line. Monoclonal antibodies or antigen binding fragments can be obtained recombinantly using, for example, hybridoma technology, recombinant techniques, phage display technology, synthetic techniques (e.g., CDR-grafting), or other prior art techniques.

When applying to an animal, human, experimental subject, cell, tissue, organ or biological fluid, 'administration' and 'treatment' refer to contacting an exogenous drug, therapeutic agent, diagnostic agent or composition with animal, human, subject, cell, tissue, organ or biological fluid. 'Administration' and 'treatment' can refer to, for example, therapeutic, pharmacokinetic, diagnostic, research, and experimental methods. Treatment of the cells includes contacting reagents with the cells, as well as contacting reagents with the fluid, wherein the fluids are in contact with the cells. 'Administration' and 'treatment' also means treating, for example, cells *in vitro* and *ex vivo* by reagents, diagnostics, binding compositions, or by another cell. 'Treatment', as it applies to a human, veterinary or research subject, refers to therapeutic treatment, prophylactic or preventative measures, to research and diagnostic applications.

'Therapy' means the administration of a therapeutic agent for internal or external use, such as a composition comprising any of the binding compounds of the present invention, to a patient having one or more symptoms of the disease for which the therapeutic agent is known to have therapeutic effect. Generally, the therapeutic agent is administered in an amount that effectively alleviates the symptoms of one or more diseases in a subject or population to be treated, whether by inducing degeneration of such symptoms or inhibiting the progression of such symptoms to any clinical measurable degree. The amount of therapeutic agent (also referred to as 'therapeutically effective amount')effective in alleviating the symptoms of any particular disease can vary depending on a variety of factors, such as disease state, age and weight of the patient, and the ability of the drug to elicit a desired effect in the patient. Whether the symptoms of the disease have been alleviated can be assessed by any clinical test method commonly used by a physician or other health care professionals to assess the severity or progression of the condition. Although embodiments of the invention (e.g., therapeutic methods or preparations) may not be effective in ameliorating the symptoms of the target disease common to each patient, it is determined that the symptoms of target disease should be alleviated in a statistically significant number of patients according to any statistical test methods known in the art such as Student's t-test, chi-square test, U test based on Mann and Whitney, Kruskal-Wallis test (H test), Jonckheere-Terpstra test, and Wilcoxon test.

The term 'consisting essentially of' or variations thereof includes all such elements or groups of elements, and optionally includes other elements that are similar or different in nature to said elements, the other elements do not significantly alter the essential or novel properties of a given dosage regimen, method or composition. As a non-limiting example, a binding compound consisting essentially of the amino acid sequence recited may also include one or more amino acids that do not significantly affect the properties of the binding compound.

The term 'naturally occurring' refers to the fact that the object can be found in nature. For example, a polypeptide sequence or a polynucleotide sequence that is present in an organism (including viruses) which can be isolated from a natural source and has not been intentionally artificially modified in the laboratory is naturally occurring.

An 'effective amount' includes an amount sufficient to ameliorate or prevent a symptom or sign of a medical condition. An effective amount also means an amount sufficient to allow or facilitate the diagnosis. An effective amount for a particular patient or veterinary subject can vary depending on factors such as the condition to be treated, the overall health of the patient, the route and dosage of the method of administration, and the severity of the side effects. An effective amount can be the maximum dose or dosing regimen that avoids significant side effects or toxic effects.

'Exogenous' refers to a substance that is produced outside of a living organism, cell, or human body according to the background. 'Endogenous' refers to a substance produced in a cell, organism or human body according to the background.

The expressions 'cell', 'cell line' and 'cell culture' as used herein can be used interchangeably and all such names include their progeny. Thus, the words 'transformant' and 'transformed cells' include primary test cells and cultures derived therefrom, regardless of the number of transfers. It should also be understood that all progeny may not be exactly identical in terms of DNA content due to intentional or unintentional mutations. Mutant progeny having the same function or biological activity as those screened in the originally transformed cell are included. When different names are meant, they are clearly distinguishable from the context.

'Optional' or 'optionally' means that the event or environment described subsequently may but does not necessarily occur, including where the event or environment occurs or does not occur. For example, 'optionally comprising 1-3 antibody heavy chain variable regions' means that the antibody heavy chain variable region of a particular sequence may, but need not, be present.

'Pharmaceutical composition' means a mixture comprising one or more of the compounds described herein, or a physiologically/pharmaceutically acceptable salt or prodrug thereof, and other chemical components, as well as other components such as physiological/pharmaceutically acceptable carriers and excipients. The purpose of the pharmaceutical composition is to promote the administration of the organism, which facilitates the absorption of the active ingredient and thereby exerts biological activity.

## Detailed description of the preferred embodiment

The following examples further illustrate the present invention and should not be construed to limit the present invention. Experimental methods without indicating specific conditions in the embodiments of the invention are usually carried out according to conventional conditions, such as Using Antibodies: A Laboratory Manualor Molecular Cloning: A Laboratory Manualof Cold Spring Harbor Laboratory; or according to the conditions recommended by the manufacturer of raw material or commodity. Reagents without specified source are routine reagents purchased from the market.

**Example 1 Screening and Discovery of human CD27 antigen-binding antibody fragments (Fab)**

*In vitro* phagemid display system was used for the screening of humanized antibody fragments against human CD27 antigen, comprising CD27-HisFc (Sino Biological #10039-H03H), CD27-Fc (Sino Biological #10039-H31H) and CD27-His (Sino Biological #10039-H08B1). The antigen was directly adsorptive immobilized on the Maxisorp 96-well screening plate (Thermo Scientific 446469), or biotinylated and adsorbed on the Dynabeads microparticles (M280, Streptavidin, Invitrogen #60210), and the KingFisher (Thermo Scientific) automatic screening workstation was used for screening upon the standard phage display screening procedures. More than 3-4 rounds of screening were conducted for each antigen, and human antibody Fc fragments were used in each round to eliminate background. After the final round of screening, single colonies were picked for cloning, incubation, and then supernatant was taken, and antibody fragments that bind to human CD27 were preliminarily identified by the ELISA method. The criterion of primary selection was that the ELISA reading was at least twice the background signal.

**Example 2 Biofilm layer interference (BLI) determination of the affinity of the antibody fragments to human CD27**

The antibody Fab fragment of each unique sequence with addition of histidine tag at its C-terminus was overexpressed using *E. coli* expression system, and then purified by Ni-NTA affinity resin. The affinity of the Fabs to human CD27 were determined using ForteBio Octet RED96 system by the following method: first the AHC (specific for human IgG antibody Fc fragment) sensor of the BLI system was used to capture the human CD27-HisFc fusion protein, and then it was immersed in a measurement buffer with the concentration of 5-10μg/ml of each purified Fab protein. The collected data was processed by Data Acquisition 7.1 software and fitted to a 1:1 Langmuir model curve. The antibody mAb077 with the best binding capacity was obtained by screening for further analysis and development experiments. The binding constant and kinetic constant of antibody to CD27 are shown in Table 1.

Table 1

| Antibody | Antigen | Affinity $K_D$(M) | Association rate $k_a$(1/M*s) | Dissociation rate $k_d$ (1/s) |
|---|---|---|---|---|
| mAb077-Fab | Human CD27-HisFc | 4.08E-09 | 2.96E+05 | 1.21E-03 |
| mAb077-IgG | Human CD27-HisFc | 1.37E-10 | 1.58E+05 | 2.17E-05 |
| 1F5-IgG | Human CD27-His | 1.39E-09 | 6.58E+04 | 9.15E-05 |
| mAb077-IgG | Murine CD27-HisFc | 3.04E-11 | 4.38E+05 | 1.33E-05 |
| 1F5-IgG | Murine CD27-HisFc | No binding | No binding | No binding |
| mAb077-IgG | MacacaCD27-Fc | 5.62E-10 | 7.58E+04 | 4.26E-05 |
| 1F5-IgG | Macaca CD27-Fc | 2.13E-09 | 1.14E+05 | 2.43E-04 |

1F5-IgG is antibody Varlilumab.

The heavy chain variable region sequence and light chain variable region sequence of human monoclonal antibody mAb077 are as follows:

mAb077 VH

EVQLVESGGGLVQPGGSLRLSCAASGFTFSGYGIHWVRQAPGKGLE WIGWINPNRGSTKYAQKFQGRVTISRDNSKNTLYLQLNSLRAEDTAVYYCAR DPGYTWYFDVWGQGTLVTVSS

SEQ ID NO:1

mAb077 VL

DIQLTQSPSSLSASVGDRVTITCRASQDISSDLAWYQQKPGKAPKLLIY AASTLQSGVPSRFSGSGSGTDFTLTISSLQPEDFATYYCQQRDAWPPTFGQGTK VEIK

SEQ ID NO:2

which contains the following CDR sequences:

| Name | Sequence | No. |
|---|---|---|
| HCDR1 | GYGIH | SEQID NO: 3 |
| HCDR2 | WINPNRGSTKYAQKFQG | SEQID NO: 4 |
| HCDR3 | DPGYTWYFDV | SEQID NO: 5 |
| LCDR1 | RASQDISSDLA | SEQID NO: 6 |
| LCDR2 | AASTLQS | SEQID NO: 7 |

(continued)

| Name | Sequence | No. |
|------|----------|-----|
| LCDR3 | QQRDAWPPT | SEQID NO: 8 |

The selected antibody light chain variable region sequence and heavy chain variable region sequence were aligned against the database. The sequence of SEQ ID NO: 1 was aligned against the heavy chain germline sequences in the database, which shows the similarity to the $V_H3$ -74 germline sequence, belonging to the $V_H3$ family of germline sequences. The sequence of SEQ ID NO: 2 was aligned against the light chain germline sequences in the database, which shows the similarity to the Vκ1-12 germline sequence, belonging to the Vκ1 family of germline sequences.

VH3-74 germline sequence

EVQLVESGGGLVQPGGSLRLSCAASGFTFSSYWMHWVRQAPGKGLV
WVSRINSDGSSTSYADSVKGRFTISRDNAKNTLYLQMNSLRAEDTAVYYCAR

SEQ ID NO:13

Vκ1-12 germline sequence

DIQMTQSPSSVSASVGDRVTITCRASQGISSWLAWYQQKPGKAPKLLI
YAASSLQSGVPSRFSGSGSGTDFTLTISSLQPEDFATYYCQQANSFP

SEQ ID NO:14

**Example 3 Conversion of antibody Fab fragments to IgG, and the expression and purification of the IgG**

Each antibody fragment was cloned into an expression vector of mammalian cell to express the light chain and heavy chain IgG1 subtype antibody. HEK293 cells were transfected with the matched vector in a transient manner according to the standard procedure. After expression, the supernatant was collected by centrifugal filtration, and then IgG was purified by protein A affinity chromatography. The eluted protein was neutralized and transferred into PB buffer (20 mM sodium phosphate, 150 mM NaCl, pH 7.0) through ion exchange. The protein concentration was measured by an ultraviolet spectrophotometer, and its purity and properties were measured under denaturing, reducing or non-reducing conditions.

The full-length sequence of light chain

mAb077 LC

DIQLTQSPSSLSASVGDRVTITCRASQDISSDLAWYQQKPGKAPKLLIY
AASTLQSGVPSRFSGSGSGTDFTLTISSLQPEDFATYYCQQRDAWPPTFGQGTK
VEIKRTVAAPSVFIFPPSDEQLKSGTASVVCLLNNFYPREAKVQWKVDNALQS
GNSQESVTEQDSKDSTYSLSSTLTLSKADYEKHKVYACEVTHQGLSSPVTKSF
NRGEC

SEQ ID NO: 9

The full-length sequence of heavy chain

mAb077 HC

EVQLVESGGGLVQPGGSLRLSCAASGFTFSGYGIHWVRQAPGKGLE
WIGWINPNRGSTKYAQKFQGRVTISRDNSKNTLYLQLNSLRAEDTAVYYCAR

DPGYTWYFDVWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICN
VNHKPSNTKVDKKVEPKSCDKTHTCPPCPAPELLGGPSVFLFPPKPKDTLMIS
RTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKL
TVDKSRWQQGNVFSCSVMHEALHNHYTQKSLSLSPGK

SEQ ID NO: 10

**Example 4 Peptide map analysis of mAb077 molecule using mass spectrum**

Preparative scale IEC was used to separate the components of acid peak and main peak of mAb077 for following testing. 500μg of each antibody sample was added with 400μL of 0.25M Tris-HCl buffer containing 6M guanidine hydrochloride and mixed well, 1M DTT was added into the samples to a final concentration of 20mM, mixed well, and incubated in water bath at 37°C for 1 hour. The samples were equilibrated to room temperature, IAM was added to a final concentration of 50mM, and the samples were incubated for 30 minutes in the dark, then transferred to a 10kDa ultrafiltration tube and centrifuged at 13000rpm for 10min. Then the samples were added with 400μl of 50 mM Tris-HCl (pH7.4) solution containing 2M urea for ultrafiltration to replace the original buffer, and the replacement of the above buffer was repeated once. The ultra-filtered samples were tested for protein concentration, and the concentration of the samples were adjusted to 0.5 mg/ml with a 50 mM Tris-HCl solution containing 2M urea. 4 μL of Trypsin enzyme was added at the ratio of sample to enzyme of 25:1, and the mixture was incubated in water bath at 37°C for 4 hours. The prepared samples were run using Acquity UPLC_Q-TOF color-mass spectrometer with ACQUITY UPLC BEH C18 column for LC-MS data collection. Chromatographic mobile phase A: 0.1% formic acid water; mobile phase B: 0.1% formic acid acetonitrile. Waters UNIFI was used to extract the mass spectrum information of each peptide, and sequences comparison was performed to determine the peptide coverage of the antibodies. The modification settings were as follows: G0F (+1444.53387) and other glycosylation settings were set to dynamic modification; Carbamidomethyl C (+57.02146) was set to fixed modification; Oxidation M and Deamidation N were dynamic modification; Target match tolerance was set to 20ppm; Fragment match tolerance was set to 50ppm.

$$\text{Deamination \%} = \frac{\text{Amino acids response to deamination modification}}{\text{All amino acid responses containing this site}} \times 100\%$$

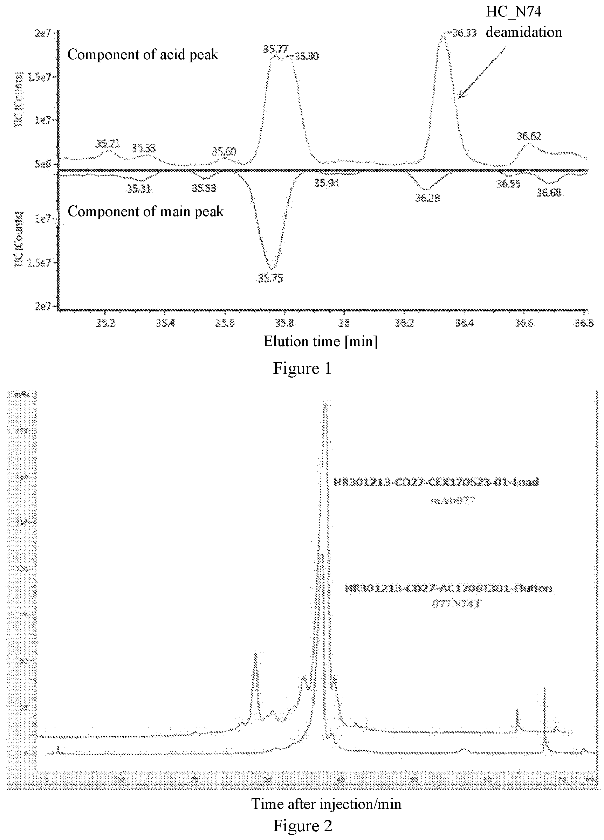

The analysis results are shown in Figure 1. The deamidation ratio of the component of acid peak of the 74th amino acid asparagine of the heavy chain (Kabat position number is 73) after separation of mAb077 was 59.6%, while the deamidation ratio of the component of main peak was only 12.2%, which confirmed that the 74th amino acid asparagine (Kabat position number is 73) in the heavy chain variable region of the mAb077 sample was deaminated significantly, and the deamidation at this site resulted in a free component of acid peak on the IEC. Therefore, we substituted the 74th amino acid asparagine (Asn) (Kabat position number is 73) of the heavy chain variable region with threonine (Thr). After the mutation, the sequence of the heavy chain variable region of molecule 077N74T is as follows (The light chain is the same as the original mAb077):

077N74T VH

EVQLVESGGGGLVQPGGSLRLSCAASGFTFSGYGIHWVRQAPGKGLE
WIGWINPNRGSTKYAQKFQGRVTISRDTSKNTLYLQLNSLRAEDTAVYYCAR
DPGYTWYFDVWGQGTLVTVSS

SEQ ID NO: 11

The full-length sequence of heavy chain of 077N74T antibody after mutation

077N74T HC

EVQLVESGGGGLVQPGGSLRLSCAASGFTFSGYGIHWVRQAPGKGLE
WIGWINPNRGSTKYAQKFQGRVTISRDNSKNTLYLQLNSLRAEDTAVYYCAR
DPGYTWYFDVWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICN
VNHKPSNTKVDKKVEPKSCDKTHTCPPCPAPELLGGPSVFLFPPKPKDTLMIS
RTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKL
TVDKSRWQQGNVFSCSVMHEALHNHYTQKSLSLSPGK

SEQ ID NO: 12

**Example 5 Biacore comparison of the affinity of anti-CD27 antibody and its mutant molecule with human CD27 antigen**

The protein A biosensor chip was used to affinity capture the antibody molecule to be tested, and then the human CD27-his antigen (Yiqiao Shenzhou# 10039-H08H) of various gradient concentrations was flowed on the surface of the chip, and the reaction signal was detected in real time with the Biacore T200 instrument to obtain the association and dissociation curve. After the dissociation of each experimental cycle was completed, the biosensor chip was washed and regenerated with glycine-hydrochloric acid regeneration solution (pH 1.5). BIAevaluation version 4.1 and GE software were used to fit the data with a (1:1) Langmuir model, the affinity values were obtained as shown in the table below. The affinity of the mutant molecule 077N74T is slightly higher than that of the original mAb077 molecule but there is no significant difference.

Table 2

| Stationary phase | Mobile phase | Association rate (1/Ms) | Dissociation rate (1/s) | Affinity (M) |
|---|---|---|---|---|
| 077N74T | huCD27-his | 2.59E5 | 6.75E-5 | 2.61E-10 |
| mAb077 | (Sino Biological) | 2.49E5 | 8.34E-5 | 3.35E-10 |

**Example 6 Analysis of ion exchange acid-base peaks of anti-CD27 antibody and its mutant molecules**

Agilent-1260 high performance liquid chromatograph was used with mobile phase A: 20 mM HEPES (pH 7.50), and mobile phase B: 20 mM HEPES + 500 mM NaCl (pH 7.50). After the antibody protein samples to be tested were desalted, the mobile phase A was used to dilute the test product to a final concentration of approximately 5.0 mg/mL, and the injection volume was 50 $\mu$g with a flow rate of 0.8 mL/min. The blank buffer and the test product were placed in the sample bottle or the lined tube respectively, and the system was balanced with 96.0% mobile phase A + 4.0% mobile phase B at a flow rate of 0.8 mL/min until the baseline was stable. Then the samples were injected, and the chromatogram was recorded. After the samples detection was finished, 1 M NaCl was used to clean the column at a flow rate of 0.2-0.5 mL/min. The analysis result is shown in Figure 2. The anti-CD27 antibody mAb077 produced a significant acid peak component at about 29 minutes in the IEC liquid phase diagram, while the component of such acid peak of mutant 077N74T molecule disappeared because the molecule did not deamidate.

**Example 7 Anti-CD27 antibody 077N74T accelerated thermal stability test**

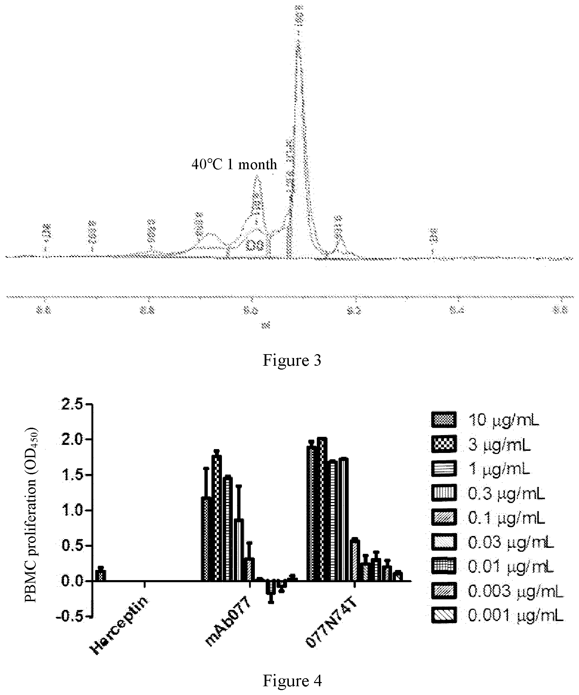

The 077N74T antibody solution was placed in an incubator at 40°C for accelerated thermal stability test. Samples were taken at time points of the 15th and 30th day, and iCIEF-full-column imaging capillary focused electrophoresis (ProteinSimple E50634 full-column imaging capillary isoelectric focusing analyzer; focusing time 1: 1500V for 1 minute; focusing time 2: 3000V for 8 minutes) was used to analyze the isoelectric point (pI) heterogeneity of the antibody protein to be tested and the trend of change of their acid-base components distribution at different time points before and after heating. The results are shown in Figure 3 and Table 3. After 077N74T was placed at 40°C for 15 days, there was no significant change in the proportions of the component; after the proteins were placed for one month, the acidic component of pI 9.0 increased by 10.4%, and the percentage of neutral group of pI 9.1 dropped by 12.3%. Overall, it shows that the mutated antibody also has good thermal stability and will not further increase the sharp acid peak due to deamidation.

Table 3

| Sampling time | ICE% | | | Main peak |
|---|---|---|---|---|
| | Acid peak | Main peak | Base peak | |
| D0 | 40.4 | 56.0 | 3.6 | / |
| 40°CD15 | 40.6 | 55.4 | 4.0 | 0.6 |
| 40°CD30 | 51.0 | 43.7 | 5.2 | 12.3 |

**Example 8 *In vitro* PBMC activation function of anti-CD27 antibody and its mutant molecules**

Human blood PBMC were separated from fresh heparin anticoagulated blood by Ficoll density gradient centrifugation. 3 $\mu$g/mL anti-CD3 antibody and different concentrations of each antibody drug to be tested (Herceptin as IgG1 subtype control) were coated in an ELISA plate and incubated overnight at 4°C. After washing the plate the next day, $2\times10^5$/well of peripheral blood mononuclear cells (PBMC) were added, and the plate was incubated in an incubator for 7 days at 37°C, with 5% $CO_2$. After the incubation, the CCK-8 kit was used to detect the proliferation of PBMC, and the $EC_{50}$ was calculated based on the $OD_{450}$ value. The result is shown in Figure 4, the stimulation of mutant molecule 077N74T for PBMC proliferation activity is slightly stronger than that of the original mAb077 molecule.

**Example 9 Comparison of *in vivo* efficacy of anti-CD27 antibody and its mutant molecules**

54 CB17-SCID mice, female, 5-6 weeks, 18-20g, purchased from Beijing Weitonglihua, were divided into 6 groups with 9 mice in each group. Raji cells were cultured *in vitro* in RPMI-1640 medium containing 10% fetal bovine serum (FBS). The Raji cells in the exponential growth phase were collected and transplanted into CB17-SCID mice subcutaneously under aseptic conditions, and each mouse was inoculated with $2\times10^6$ cells. The reference antibody Varlilumab is an anti-CD27 antibody developed by Celldex (see CN103154034B for details). The day of tumor inoculation was day 0. The first administration was performed on the third day of vaccination, and then proceeded as follows:

Group 1: IgG1 30mg/kg, i.p., N=9, Q2D, 3, 5, 7, 9, 11, 13, 15, 17 days;

Group 2: 077N74T 3mg/kg, i.p., N=9, Q2D, 3, 5, 7, 9, 11, 13, 15, 17 days;

Group 3: 077N74T 10mg/kg, i.p., N=9, Q2D, 3, 5, 7, 9, 11, 13, 15, 17 days;

Group 4: 077N74T 30mg/kg, i.p., N=9, Q2D, 3, 5, 7, 9, 11, 13, 15, 17 days;

Group 5: mAb077 30mg/kg, i.p., N=9, Q2D, 3, 5, 7, 9, 11, 13, 15, 17 days;

Group 6: Varlilumab 30mg/kg, i.p., N=9, Q2D, 3, 5, 7, 9, 11, 13, 15, 17 days;

The daily behavior of the animals was monitored every day after administration for 18 days. After measuring the long diameter and short diameter of the tumor with vernier calipers, the tumor volume was calculated with length×width$^2$/2. The Tumor Growth Inhibition value adopted the formula TGI%=(1-T/C)×100%. T and C are the tumor volume (TV) of the treatment group and the control group at a specific time point, respectively. The weight change adopted the formula RCBW%=(BWi-BW0) / BWO×100%, BWi is the current weight of the mouse, and BW0 is the weight of the mouse on the day of grouping. All experimental results were indicated as mean tumor volume ± standard error (SEM). The T-Test method was used to compare the tumor volume and tumor weight between the treatment group and the control group to see whether there are significant differences. All data were analyzed by Graphpad, and p<0.05 indicated significant difference.

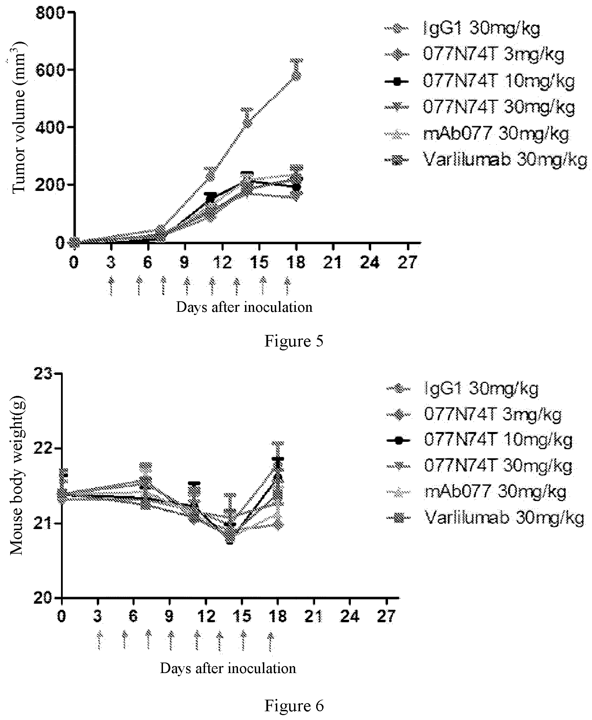

As shown in Figure 5 and Figure 6, in the Raji transplanted tumor model, 077N74T showed an anti-tumor effect with dose-dependent. The Tumor Growth Inhibition value (TGI) of the low, medium and high dose groups were 62%, 67% and 73%, respectively. And the anti-tumor effect of 077N74T in the highest dose group (30mg/kg) was slightly stronger than that of mAb077 before mutation (59%) or the reference antibody Varlilumab (61%) at the same dose. There was no significant weight loss of mice in each administration group, indicating that the mice tolerated each antibody at different doses well.

Although specific embodiments of the present invention have been described above, those skilled in the art should understand that these are merely illustrative, and various changes or modifications can be made to the present invention without departing from the principles and spirits of the present invention, and these equivalent forms also fall within the scope defined by the appended claims of the present application.

**Claims**

1. An anti-CD27 antibody or an antigen-binding fragment thereof, comprising: a light chain variable region and a heavy chain variable region, wherein the light chain variable region of the antibody comprises amino acid sequences of LCDR1, LCDR2 and LCDR3 shown in SEQ ID NO: 6,SEQ ID NO: 7 and SEQ ID NO: 8, respectively; and/or, the heavy chain variable region of the antibody comprises amino acid sequences of HCDR1, HCDR2 and HCDR3 shown in SEQ ID NO: 3, SEQ ID NO: 4 and SEQ ID NO: 5, respectively.

2. The anti-CD27 antibody or the antigen-binding fragment thereof of claim 1, wherein the anti-CD27 antibody or antigen-binding fragment further comprises a framework region of Vκ1 sequence of human germline light chain variable region or a derivative sequence thereof; preferably, the framework region of the light chain variable region is derived from Vκ1-12sequence;
and/or, the anti-CD27 antibody or the antigen-binding fragment thereof further comprises a framework region of $V_H3$ sequence of human germline heavy chain variable region or a derivative sequence thereof; preferably, the framework region of the heavy chain variable region sequence is derived from $V_H3$-74sequence.

3. The anti-CD27 antibody or the antigen-binding fragment thereof of claim 1 or 2, wherein an asparagine at position 73 of Kabat in the framework region sequence of the $V_H3$ sequence of the heavy chain variable region of the antibody is substituted by a threonine.

4. The anti-CD27 antibody or the antigen-binding fragment thereof of any one of the preceding claims, wherein the amino acid sequence of the light chain variable region is shown in SEQ ID NO: 2 or has at least 90% identity with SEQ ID NO: 2; and/or the amino acid sequence of the heavy chain variable region is shown in SEQ ID NO: 1 or SEQ ID NO: 11, or has at least 90% identity with SEQ ID NO: 1 or SEQ ID NO: 11.

5. The anti-CD27 antibody or the antigen-binding fragment thereof of any one of the preceding claims, which is a human antibody or a fragment thereof.

6. The anti-CD27 antibody or the antigen-binding fragment thereof of any one of the preceding claims, wherein the heavy chain of the human antibody comprises a heavy chain constant region of human IgG1, IgG2, IgG3 or IgG4 or a variant thereof, and the antigen-binding fragment is a Fab, Fab'-SH, Fv, scFv or (Fab')$_2$ fragment; preferably, the heavy chain of the human antibody comprises a heavy chain constant region of human IgG1, IgG2 or IgG4.

7. The anti-CD27 antibody or the antigen-binding fragment thereof of any one of the preceding claims, wherein the amino acid sequence of the light chain of the anti-CD27 antibody or antigen-binding fragment thereof is shown in SEQ ID NO: 9 or has at least 90% identity with SEQ ID NO: 9; and/or the amino acid sequence of the heavy chain of the anti-CD27 antibody or the antigen-binding fragment thereof is shown in SEQ ID NO: 10 or SEQ ID NO: 12, or has at least 90% identity with SEQ ID NO: 10 or SEQ ID NO: 12.

8. A polynucleotide encoding the anti-CD27 antibody or the antigen-binding fragment thereof of any one of claims 1-7.

9. A vector comprising the polynucleotide of claim 8, wherein the vector is a eukaryotic expression vector, a prokaryotic expression vector or a viral vector.

10. A host cell obtained through transformation with the vector of claim 9; preferably, the host cell is a bacteria, yeast or mammalian cell; more preferably, the host cell is an *Escherichia coli, Pichia pastoris,* Chinese hamster ovary cell or human embryonic kidney 293 cell.

11. A method for preparing the anti-CD27 antibody and the antigen-binding fragment thereof of any one of claims 1-7, wherein the method comprises expressing the antibody or the antigen-binding fragment thereof in the host cell of claim 10, and isolating the antibody and the antigen-binding fragment thereof.

12. An antibody-drug conjugate, wherein the antibody comprises the anti-CD27 antibody or the antigen-binding fragment thereof of any one of claims 1-7.

13. A pharmaceutical composition comprising the anti-CD27 antibody or the antigen-binding fragment thereof of any one of claims 1-7, and a pharmaceutically acceptable excipient, dilution or carrier.

14. A use of the anti-CD27 antibody or antigen-binding fragment thereof of any one of claims 1-7, or the antibody-drug conjugate of claim 12, or the pharmaceutical composition of claim 13 in the preparation of medicament for the treatment and/or prevention of a CD27-mediated disease or disorder; preferably, the disease or disorder is a cancer or an autoimmune disease; more preferably, the disease or disorder is a cancer or an autoimmune disease with CD27 expression; more preferably, the cancer is rectal cancer, brain cancer, renal cancer, lung cancer, liver cancer, multiple myeloma and melanoma, and the autoimmune disease is autoimmune encephalomyelitis, systemic lupus erythematosus, multiple sclerosis and rheumatoid arthritis.

15. A method for the treatment and/or prevention of a CD27-mediated disease or disorder, wherein the method comprises administering to a patient in need a therapeutically effective amount of the anti-CD27 antibody or the antigen-binding fragment thereof of any one of claims 1-7, or the antibody-drug conjugate of claim 12, or the pharmaceutical composition of claim 13; preferably, the disease or disorder is a cancer or an autoimmune disease; more preferably, the disease or disorder is a cancer or an autoimmune disease expressing CD27; more preferably, the cancer is rectal cancer, brain cancer, renal cancer, lung cancer, liver cancer, multiple myeloma and melanoma, and the autoimmune disease is autoimmune encephalomyelitis, systemic lupus erythematosus, multiple sclerosis and rheumatoid arthritis.

Figure 1

Time after injection/min

Figure 2

40°C 1 month

Figure 3

Figure 4

Figure 5

Figure 6

## INTERNATIONAL SEARCH REPORT

| | International application No. |
|---|---|
| | **PCT/CN2019/079811** |

**A. CLASSIFICATION OF SUBJECT MATTER**

C07K 16/28(2006.01)i; C12N 15/09(2006.01)i; C12N 5/10(2006.01)i; C12N 1/21(2006.01)i; C12N 1/15(2006.01)i; C12P 21/08(2006.01)i; A61K 39/395(2006.01)i; A61P 35/00(2006.01)i; A61P 37/00(2006.01)i

According to International Patent Classification (IPC) or to both national classification and IPC

**B. FIELDS SEARCHED**

Minimum documentation searched (classification system followed by classification symbols)

C07K; C12N; C12P; A61K; A61P

Documentation searched other than minimum documentation to the extent that such documents are included in the fields searched

Electronic data base consulted during the international search (name of data base and, where practicable, search terms used)

CPRSABS; CNABS; CNKI; CNTXT; DWPI; SIPOABS; EPTXT; USTXT; WOTXT; JPTXT; ISI Web of Science; CA: CD27, 抗体, 人源化, Vκ, VH, cluster of differentiation 27, antibody, humanized等; 中国专利生物序列检索系统; China Patents Biological Sequence Search System; Genbank; EMBL; STN和检索的序列: SEQ ID NOs: 1-14等, STN and searched sequence: SEQ ID NOs: 1-14 et al.

**C. DOCUMENTS CONSIDERED TO BE RELEVANT**

| Category* | Citation of document, with indication, where appropriate, of the relevant passages | Relevant to claim No. |
|---|---|---|
| PX | WO 2018059465 A1 (JIANGSU HENGRUI MEDICINE CO., LTD.) 05 April 2018 (2018-04-05) see claims 1-35 | 1-14 |
| A | CN 104284678 A (JANSSEN BIOTECH, INC.) 14 January 2015 (2015-01-14) see entire document | 1-14 |
| A | CN 105669865 A (CELLDEX THERAPEUTICS INC) 15 June 2016 (2016-06-15) see entire document | 1-14 |
| A | CN 102007147 A (KYOWA HAKKO KIRIN CO., LTD.) 06 April 2011 (2011-04-06) see entire document | 1-14 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **03 June 2019** | **27 June 2019** |

| Name and mailing address of the ISA/CN | Authorized officer |
|---|---|
| **State Intellectual Property Office of the P. R. China (ISA/ CN)** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

## INTERNATIONAL SEARCH REPORT

| | |
|---|---|
| International application No. | |
| **PCT/CN2019/079811** | |

**Box No. I          Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

　　a.  ☑ forming part of the international application as filed:

　　　　　☑ in the form of an Annex C/ST.25 text file.

　　　　　☐ on paper or in the form of an image file.

　　b.  ☐ furnished together with the international application under PCT Rule 13*ter*.1(a) for the purposes of international search only in the form of an Annex C/ST.25 text file.

　　c.  ☐ furnished subsequent to the international filing date for the purposes of international search only:

　　　　　☐ in the form of an Annex C/ST.25 text file (Rule 13*ter*.1(a)).

　　　　　☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**

International application No.

**PCT/CN2019/079811**

**Box No. II      Observations where certain claims were found unsearchable (Continuation of item 2 of first sheet)**

This international search report has not been established in respect of certain claims under Article 17(2)(a) for the following reasons:

1. ☑  Claims Nos.: **15**
       because they relate to subject matter not required to be searched by this Authority, namely:

   [1]   PCT Rule 39.1(iv)—methods for treatment of the human body or animal body by surgery or therapy.

2. ☐  Claims Nos.:
       because they relate to parts of the international application that do not comply with the prescribed requirements to such an
       extent that no meaningful international search can be carried out, specifically:

3. ☐  Claims Nos.:
       because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.

**PCT/CN2019/079811**

| Patent document cited in search report | | | Publication date (day/month/year) | Patent family member(s) | | | Publication date (day/month/year) |
|---|---|---|---|---|---|---|---|
| WO | 2018059465 | A1 | 05 April 2018 | CN | 108473586 | A | 31 August 2018 |
| | | | | TW | 201813979 | A | 16 April 2018 |
| CN | 104284678 | A | 14 January 2015 | ZA | 201500887 | B | 27 January 2016 |
| | | | | US | 2017320957 | A1 | 09 November 2017 |
| | | | | US | 9683046 | B2 | 20 June 2017 |
| | | | | US | 2013243795 | A1 | 19 September 2013 |
| | | | | UY | 34680 | A | 30 September 2013 |
| | | | | PE | 22422014 | A1 | 08 January 2015 |
| | | | | JP | 6487839 | B2 | 20 March 2019 |
| | | | | CR | 20140415 | A | 18 November 2014 |
| | | | | US | 9102737 | B2 | 11 August 2015 |
| | | | | JP | 2015511965 | A | 23 April 2015 |
| | | | | NZ | 629697 | A | 27 January 2017 |
| | | | | US | 2015299330 | A1 | 22 October 2015 |
| | | | | CO | 7071095 | A2 | 30 September 2014 |
| | | | | EP | 2825200 | A4 | 26 August 2015 |
| | | | | ZA | 201407440 | B | 27 July 2016 |
| | | | | HK | 1206251 | A1 | 08 January 2016 |
| | | | | CN | 104284678 | B | 24 April 2018 |
| | | | | PH | 12014502011 | B1 | 24 November 2014 |
| | | | | SG | 11201405437Q | A | 30 October 2014 |
| | | | | AU | 2013232087 | B2 | 08 February 2018 |
| | | | | EP | 2825200 | A1 | 21 January 2015 |
| | | | | EA | 030828 | B1 | 31 October 2018 |
| | | | | PH | 12017501908 | A1 | 01 October 2018 |
| | | | | TW | 201730214 | A | 01 September 2017 |
| | | | | GT | 201400193 | A | 14 June 2017 |
| | | | | SG | 10201710574U | A | 27 February 2018 |
| | | | | MX | 2014011100 | A | 05 December 2014 |
| | | | | TW | 201345922 | A | 16 November 2013 |
| | | | | CA | 2867299 | A1 | 19 September 2013 |
| | | | | TW | I576354 | B | 01 April 2017 |
| | | | | EA | 201491700 | A1 | 27 February 2015 |
| | | | | AR | 090356 | A1 | 05 November 2014 |
| | | | | AU | 2013232087 | A1 | 25 September 2014 |
| | | | | PH | 12014502011 | A1 | 24 November 2014 |
| | | | | WO | 2013138586 | A1 | 19 September 2013 |
| | | | | BR | 112014022812 | A2 | 18 July 2017 |
| | | | | KR | 20140133940 | A | 20 November 2014 |
| | | | | CL | 2014002416 | A1 | 16 January 2015 |
| CN | 105669865 | A | 15 June 2016 | US | 2015337047 | A1 | 26 November 2015 |
| | | | | US | 2011274685 | A1 | 10 November 2011 |
| | | | | KR | 20130066605 | A | 20 June 2013 |
| | | | | EP | 3165540 | A1 | 10 May 2017 |
| | | | | US | 9169325 | B2 | 27 October 2015 |
| | | | | NZ | 623807 | A | 31 March 2016 |
| | | | | ES | 2608475 | T3 | 11 April 2017 |
| | | | | CA | 2796571 | A1 | 20 October 2011 |
| | | | | EP | 2558498 | A2 | 20 February 2013 |
| | | | | EA | 024701 | B1 | 31 October 2016 |

Form PCT/ISA/210 (patent family annex) (January 2015)

**INTERNATIONAL SEARCH REPORT**
Information on patent family members

International application No.

**PCT/CN2019/079811**

| Patent document cited in search report | | | Publication date (day/month/year) | Patent family member(s) | | | Publication date (day/month/year) |
|---|---|---|---|---|---|---|---|
| | | | | CN | 103154034 | A | 12 June 2013 |
| | | | | EA | 201690310 | A1 | 30 December 2016 |
| | | | | JP | 6002659 | B2 | 05 October 2016 |
| | | | | CN | 103154034 | B | 08 June 2016 |
| | | | | WO | 2011130434 | A3 | 08 March 2012 |
| | | | | IL | 222329 | A | 30 August 2018 |
| | | | | MX | 2012011768 | A | 09 May 2013 |
| | | | | WO | 2011130434 | A2 | 20 October 2011 |
| | | | | JP | 6486300 | B2 | 20 March 2019 |
| | | | | KR | 101958753 | B1 | 15 March 2019 |
| | | | | AU | 2011239706 | B2 | 20 August 2015 |
| | | | | JP | 2013531970 | A | 15 August 2013 |
| | | | | NZ | 602892 | A | 29 August 2014 |
| | | | | SG | 184310 | A1 | 30 October 2012 |
| | | | | EA | 201291039 | A1 | 30 April 2013 |
| | | | | IL | 222329 | D0 | 31 December 2012 |
| | | | | EP | 2558498 | B1 | 12 October 2016 |
| | | | | JP | 2017046692 | A | 09 March 2017 |
| | | | | MX | 339621 | B | 02 June 2016 |
| | | | | AU | 2011239706 | A1 | 01 November 2012 |
| | | | | WO | 2011130434 | A9 | 19 July 2012 |
| CN | 102007147 | A | 06 April 2011 | EP | 2314628 | A4 | 10 July 2013 |
| | | | | CA | 2729567 | C | 24 April 2018 |
| | | | | US | 9023999 | B2 | 05 May 2015 |
| | | | | JP | WO2010001908 | A1 | 22 December 2011 |
| | | | | EP | 2314628 | A1 | 27 April 2011 |
| | | | | JP | 5425775 | B2 | 26 February 2014 |
| | | | | AU | 2009267294 | A1 | 07 January 2010 |
| | | | | KR | 20110039220 | A | 15 April 2011 |
| | | | | CA | 2729567 | A1 | 07 January 2010 |
| | | | | AU | 2009267294 | B2 | 26 June 2014 |
| | | | | WO | 2010001908 | A1 | 07 January 2010 |
| | | | | US | 2010173324 | A1 | 08 July 2010 |
| | | | | CN | 102007147 | B | 05 November 2014 |

Form PCT/ISA/210 (patent family annex) (January 2015)

**REFERENCES CITED IN THE DESCRIPTION**

**Patent documents cited in the description**

- CN 201810267050
- WO 2008051424 A
- WO 2011130434 A
- CN 2017103842 W
- CN 103154034 B

**Non-patent literature cited in the description**

- **CAMERINI et al.** *J Immunol.,* 1991, vol. 147, 3165-69
- **KOBATA T et al.** *Proc. Natl. Acad. Sci. USA.,* 1995, vol. 24 (11249-53
- **RANHEIM EA et al.** *Blood,* 1995, vol. 85 (12), 3556-65
- **LOENEN WA et al.** *Eur. J. Immunol.,* 1992, vol. 22, 447
- *J. Biol. Chem,* 1968, vol. 243, 3558
- **TOMLINSON et al.** *J. Mol. Biol.,* 1992, vol. 227, 776-798
- **COOK et al.** *Immunology Today,* 1995, vol. 16, 237-242
- **ENRIQUE et al.** *Int Immunol.,* December 1997, vol. 9 (12), 1801-15
- **WINTER et al.** *Annual Rev. Immunol.,* 1994, vol. 12, 433-55
- Using Antibodies: A Laboratory Manual. Cold Spring Harbor
- The Immunoglobulin FactsBook. 2001
- Using Antibodies: A Laboratory Manualor Molecular Cloning: A Laboratory Manual. Cold Spring Harbor Laboratory